# EUROPEAN PATENT APPLICATION

(11) **EP 2 848 241 A1**
(43) Date of publication of application: **18.03.2015**
(21) Application number: 14184471.2
(22) Date of filing: 11.09.2014
(51) Int. Cl.: A61K 9/00, A61K 9/20, A61K 31/155

(54) **Effervescent formulations of linagliptin**

(30) Priority: 12.09.2013 TR 201310724; 24.09.2013 TR 201311199
(71) Applicant: Sanovel Ilac Sanayi ve Ticaret A.S., 34460 Istanbul (TR)
(72) Inventor: Türkyilmaz, Ali, 34460 Istanbul (TR); Turp, Ali Hasan, 34460 Istanbul (TR); Saydam, Mehtap, 34460 Istanbul (TR)
(74) Representative: Sevinç, Erkan

(57) **Abstract**

The present invention relates to an effervescent formulation comprising linagliptin or a pharmaceutically acceptable salt thereof and one or more pharmaceutically acceptable excipients.

## Description

### Field of Invention

The present invention relates to an effervescent formulation comprising linagliptin or a pharmaceutically acceptable salt thereof and one or more pharmaceutically acceptable excipients.

### Background of Invention

Linagliptin is used for type 2 or non-insulin dependent diabetes. It is a selective, orally administered, xanthine based dipeptidyl peptidase-4 (DPP-4) inhibitor used as an adjunct to diet and exercise to improve glycemic control. DPP-4 inhibitors work by blocking the action of DPP-4, an enzyme which destroys the hormone incretin. There are two types of incretin hormones found in the body, called glucagon-like peptide-1 (GLP-1) and glucose-dependent insulinotropic peptide (GIP). These hormones are naturally produced by the body in response to food intake. Their function is to help the body produce more insulin only when it is needed and reduce the amount of glucose being produced by the liver when it is not needed. Linagliptin works by binding to DPP-4 and preventing it from breaking down the GLP-1 and GIP. This increases the levels of these hormones in the body and so increases their effect on controlling blood sugar.

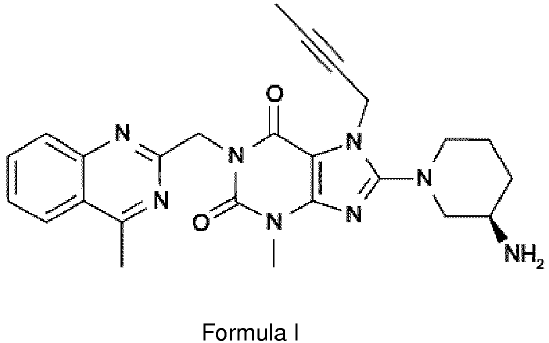

Its chemical name is 8-[(3R)-3-aminopiperidin-1-yl]-7-but-2-yn-1-yl)-3-methyl-1-[(4-methylquinazolin-2-yl)methyl]-3,7-dihydro-1H-purine-2,6-dione and its chemical structure is shown in the Formula I.

There is Linagliptin coated tablet formulation license in the US and EU under the brand name TRADJENTA^{®} by Boehringer Ingelhem International. TRADJENTA^{®} includes mannitol, prejelatinized starch, maize starch, copovidone, magnesium stearate in the tablet core and hypromellose, titanium dioxide, talc, macrogol 6000 and red iron oxide in the film coating. It is used once daily and can be used either alone or in combination with insulin or other glycemic agents.

EP 2 023 902 B1 provides pharmaceutical formulations comprising DPP-4 inhibitors included Linagliptin. It is indicated that DPP-4 inhibitors with primary amine group shows incompatibilities, degradation problems or extraction problems with excipients such as microcrystalline cellulose, sodium starch glycolate, croscarmellose sodium, tartaric acid, citric acid, glucose, fructose, saccharose, lactose, maltodextrines. Linagliptin has also a primary amine group on its chemical structure. In solid dosage forms, it may react with many excipients or impurities of excipients, although linagliptin itself is very stable. Thus, aforementioned patent provides Linagliptin tablet formulations which do not comprise above excipients.

In the state of art, there are several patents and applications which disclose tablet formulations of linagliptin but none of them comprises effervescent formulations of linagliptin.

Over the past decades, effervescent formulations have gained considerable attention as a preferred alternative to conventional tablets and capsules due to their better patient compliance. There are a growing number of people who cannot swallow tablets or capsules. Effervescent formulations are intended to be dissolved or dispersed in water before administration and liquid effervescent form is easier to take as compared to tablets or capsules.

In addition to ease of administration, many studies have demonstrated that effervescent formulations enhance absorption of a number of active ingredients. They generally contain acidifying agents and alkalizing agents such as carbonates or bicarbonates and which react rapidly in the presence of water by releasing carbon dioxide. Carbon dioxide emitted by the effervescent reaction can induce enhanced active-ingredient permeability due to an alteration of the paracellular pathway. Moreover, the low pH in the stomach can cause active ingredients to become denatured, lose activity, or cause them to remain inactive. Acidifying agent - alkalizing agent couples, however, can buffer the water-active solution so that the stomach pH increases and thus prevent the degradation or inactivation of the active ingredient.

On the other side, effervescent formulations are not easy to process. Unpleasant and bitter taste upon dissolution in water is problems that should be overcome. Moreover, these formulations should be porous and not too rigid to dissolve in water. However, this porous structure may cause stability problems by affecting from humidity. It is needed to develop an effervescent formulation with non-hygroscopic formulation.

In this invention, effervescent formulations comprising linagliptin or a pharmaceutically acceptable salt thereof and one or more pharmaceutically acceptable excipients have been developed. To overcome stability problem, one or more pharmaceutically acceptable excipient has been used.

### Description of the invention

The present invention provides an effervescent formulation comprising linagliptin or a pharmaceutically acceptable salt thereof and one or more pharmaceutically acceptable excipients.

In one embodiment, the effervescent formulation is in the form of a tablet or a sachet.

In another embodiment, the effervescent formulation of this present invention is used by directly taking into mouth or by introducing the formulation into water to be administered.

According to one embodiment, linagliptin or a pharmaceutically acceptable salt thereof is present in an amount of 2 to 80 %, preferably 3.5 to 60 % and more preferably 10 to 40 % by weight of total formulation.

According to one embodiment, said one or more pharmaceutically acceptable excipient is selected from the group comprising acidifying agent, alkalizing agent, lubricants, fillers, disintegrants, binders, antioxidants, aromatic agents and sweeteners.

In general, effervescent formulations comprise acidifying agents and alkalizing agents such as carbonates or bicarbonates and which reacts rapidly in the presence of water by releasing carbon dioxide. Choice of acidifying agent - alkalizing agent couple and their ratio is important in terms of stability, disintegration time and water solubility.

The ratio of acidifying agent to alkalizing agent should be optimum to obtain a neutral solution without any residue and precipitation after tablet disintegration. High amount of acidifying agent causes low pH which may damage the gastrointestinal system. On the other side, high amount of alkalizing agent (especially hydrophobic agents) also causes unclear and precipitated solution and induces damage in gastrointestinal system. In this invention, within a specific ratio of them, desired neutral solution without any precipitation has been achieved. Moreover, by adjusting the ratio of acidifying agent to alkalizing agent, desired taste of effervescent solution has also been obtained in order to contribute aromatic agents used in the formulation.

According to this embodiment, the ratio of the acidifying agents to the alkalizing agents is between 10:1 (w/w) and 1:10 (w/w), preferably between 5:1 (w/w) and 1:5 (w/w) and more preferably it is between 2.9:1 (w/w) and 1.1:5 (w/w).

According to this embodiment, the acidifying agent is selected from the group comprising fumaric acid, citric acid, adipic acid, acetic acid, hydrochloric acid, malic acid, nitric acid, phosphoric acid, sulfuric acid, tartaric acid and mixtures thereof, preferably it is fumaric acid.

According to this embodiment, the alkalizing agent is selected from the group comprising sodium glycine carbonate, ammonia solution, ammonium carbonate, diethanolamine, diisopropanolamine, potassium hydroxide, sodium bicarbonate, sodium borate, sodium carbonate, sodium hydroxide, trolamine and mixtures thereof, preferably it is sodium glycine carbonate.

Effervescent formulations should be porous and not too rigid to dissolve in water. However, this porous structure may cause stability problems by affecting from humidity, especially during the production, packaging or transportation. Therefore, using suitable excipients is essential to ensure the stability of the effervescent formulation.

According to this embodiment, acidifying agent - alkalizing agent couple should be non-hygroscopic to overcome stability problem. Fumaric acid has been used as an acidifying agent. In contrast to unstable acidifying agents disclosed in EP 2 023 902 B1, fumaric acid has not given rise to stability problems with linagliptin containing amine group. Moreover, it shows lubricating property, prevents stickness and improves compressibility. In addition, as an alkalizing agent, sodium glycine bicarbonate which reacts rapidly in the water by releasing carbon dioxide has been used. When Fumaric acid and sodium glycine bicarbonate have been used together, a non-hygroscopic acidifying agent - alkalizing agent couple have been obtained which shows a synergistic effect on disintegration time, stability and the production method of the formulation.

In this embodiment, the amount of fumaric acid is in the range of 2 - 90 %, preferably 5 - 60 %, more preferably it is 10 - 40 % by weight of total formulation.

In this embodiment, the amount of sodium glycine bicarbonate is in the range of 2 - 90 %, preferably 5 - 60 %, more preferably it is 10 - 40 % by weight of total formulation.

In another embodiment, suitable lubricants are selected from the group comprising polyethylene glycol (PEG), sodium stearyl fumarate, sodium lauryl sulphate, magnesium lauryl sulphate, fumaric acid, glyceryl palmitostearate, hydrogenated natural oils, zinc stearate, calcium stearate, silica, talc, stearic acid, paraffin and mixtures thereof, preferably polyethylene glycol.

In this present invention, polyethylene glycol has been chosen as a lubricant due to its distinguishing properties among other lubricants. Polyethylene glycols are biocompatible, stable, hydrophilic substances used in pharmaceutical formulations. It does not give rise to over-blending related problems, which is observed with magnesium stearate. Comparison to the conventional use, in this invention, polyethylene glycol has been used both as a lubricant and a stabilizer. As a lubricant, it does not interact with production equipment during the process and neutral solution without any precipitation has been achieved due to its hydrophilic properties. Moreover, due to its non-hygroscopic property, it has shown synergistic effect with acidifying agent - alkalizing agent couple and further ensured the stability of the effervescent formulation throughout its shelf life.

According to this embodiment, the amount of polyethylene glycol (PEG) is in the range of 0.1 to 15 %, preferably 0.1 to 10%, more preferably it is 0.1 to 2% by weight of total formulation.

Polyethylene glycol with different grades shows different properties. During the pharmaceutical production, high molecular weight polyethylene glycol can be used as a lubricant. However, very high molecular weight of polyethylene glycol has drawbacks during the process such as stickiness. Moreover, viscosity that has an effect on compressibility is increasing with the increase in molecular weight of polyethylene glycol. Another property is affected from molecular weight is hygroscopicity. Hygroscopicity increases as molecular weight decreases and this may results stability problems of active ingredient. As a result, in this invention an optimum molecular weight of polyethylene glycol has been used to provide non-hygroscopicity without stickness or compressibility problems.

According to this embodiment, molecular weight of polyethylene glycol in this formulation is in the range of 1500 - 9000 Daltons (Da), it is preferably 1500 - 5000 Da and more preferably 1600 - 4000 Da.

The stability assay is performed by HPLC at a wavelength of 225nm at 45 ºC column temperature in the presence of phosphate buffer as a mobile phase.

Suitable fillers may include but not limited to lactose, sugars, mannitol, sorbitol, sucrose, inorganic salts, calcium salts, polysaccharides, dextrose, dicalsium phosphate, sodium chloride, dextrates, lactitol, maltodextrin, sucrose-maltodextrin mixtures, xylitol, trehalose, heavy magnesium carbonate, izomalt and mixtures thereof.

Suitable disintegrants may include but not limited to alginic acid and alginates, ion-exchange resins, magnesium aluminum silicate, sodium dodecyl sulfate, sodium carboxymethyl cellulose, croscarmellose sodium, cross-linked polyvinylpyrrolidone (PVP), carboxymethyl cellulose calcium, docusate sodium, guar gum, low-substituted hydroxypropyl cellulose, polyacrylin potassium, poloxamer, povidone, sodium alginate, sodium glycine carbonate, sodium lauryl sulfate, or the mixtures thereof.

Suitable binders may include but not limited to sugars, glucose syrup, natural gums, starch, gelatin, polyvinylpyrrolidone, polymethacrylates; collagen, proteins such as gelatin; agar, alginate, sodium alginate, pectin, starch, carboxymethyl cellulose, hydroxypropyl cellulose, methyl cellulose and similar semi-synthetic polymers; carbomer, poloxamer, polyacrylamide, polyvinyl alcohol and similar synthetic polymers; aluminum hydroxide, bentonite, laponite and other inorganic substances; starch mucilage, acacia mucilage, polydextrose, polyethylene oxide, or the mixtures thereof.

Suitable antioxidants may include but not limited to butyl hydroxyanisole, butyl hydroxytoluene, ascorbic acid, beta-carotene, alpha-tocopherol, propyl gallate, gentisic acid, sodium ascorbate, sodium bisulfate, sodium metabisulfate, monothioglycerol, cysteine, sodium thioglycolate, acetone sodium bisulfite, sodium bisulfate, sodium dithionite, gentisic acid ethanolamine, monosodium glutamate, sodium formaldehyde sulfoxylate, alpha tocopherol, or the mixtures thereof.

Suitable aromatic agents may include but not limited to fruit aromas such as orange, banana, strawberry, cherry, wild cherry, lemon, etc., and other aromas such as cardamom, anis, mint, menthol, vanillin, and the mixtures thereof.

Suitable sweeteners may include but not limited to sucralose, thaumatin, mogroside, inuline, erythritol, or mixtures thereof.

In this present invention, to achieve stability with an improved process, this formulation has been designed, comprising the following:
a. 2.0 - 80.0 % by weight of linagliptin
b. 0.1 - 15.0 % by weight of polyethylene glycol
c. 2.0 - 90.0 %-by weight of sodium glycine carbonate
d. 2.0 - 90.0 % by weight of fumaric acid
e. 5.0 - 90.0 % by weight of lactose
f. 0.1 - 0.2 % by weight of sweetener
g. 0.1 - 5.0 % by weight of aromatic agent

### Example: effervescent tablet

| **ingredients** | **Amount (%)** |
|---|---|
| linagliptin | 3.5 - 60.0 |
| polyethylene glycol | 0.1 - 2.0 |
| sodium glycine carbonate | 5.0 - 60.0 |
| fumaric acid | 5.0 - 60.0 |
| lactose | 5.0 - 90.0 |
| sweetener | 0.1 - 0.2 |
| aromatic agent | 0.1 - 5.0 |

The production of the formulation is carried out as follows: Linagliptin, aromatic agent and sweetener are mixed and lactose, sodium glycine carbonate and fumaric acid are added to mixture respectively and mixed. Polyethylene glycol is added to final powder mixture and mixed. The mixture is filled into bags in sachet filling machine or pressed into tablets.

With this invention, an effervescent formulation comprising linagliptin or a pharmaceutically acceptable salt thereof is achieved which is eliminating stability, process and disintegration related problems and bringing additional advantages.

## Claims

1. An effervescent formulation comprising linagliptin or a pharmaceutically acceptable salt thereof in an amount of 2 to 80 %, preferably 3.5 to 60 % and more preferably 10 to 40 % by weight of total formulation and one or more pharmaceutically acceptable excipients.

2. The effervescent formulation according to claim 1, wherein one or more pharmaceutically acceptable excipient is selected from the group comprising acidifying agent, alkalizing agent, lubricants, fillers, disintegrants, binders, antioxidants, aromatic agents and sweeteners.

3. The effervescent formulation according to claim 3, the ratio of the acidifying agents to the alkalizing agents is between 10:1 (w/w) and 1:10 (w/w), preferably between 5:1 (w/w) and 1:5 (w/w) and more preferably it is between 2.9:1 (w/w) and 1.1:5 (w/w).

4. The effervescent formulation according to claim 3, wherein the acidifying agent is selected from the group comprising fumaric acid, citric acid, adipic acid, acetic acid, hydrochloric acid, malic acid, nitric acid, phosphoric acid, sulfuric acid, tartaric acid and mixtures thereof, preferably it is fumaric acid.

5. The effervescent formulation according to claim 5, wherein the amount of fumaric acid is in the range of 2 - 90 %, preferably 5 - 60 %, more preferably it is 10 - 40 % by weight of total formulation.

6. The effervescent formulation according to claim 3, wherein the alkalizing agent is selected from the group comprising sodium glycine carbonate, ammonia solution, ammonium carbonate, diethanolamine, diisopropanolamine, potassium hydroxide, sodium bicarbonate, sodium borate, sodium carbonate, sodium hydroxide, trolamine and mixtures thereof, preferably it is sodium glycine carbonate.

7. The effervescent formulation according to claim 7, wherein the amount sodium glycine carbonate is in the range of 2 - 90 %, preferably 5 - 60 %, more preferably it is 10 - 40 % by weight of total formulation.

8. The effervescent formulation according to claim 3, wherein the lubricant is selected from the group comprising polyethylene glycol, sodium stearyl fumarate, sodium lauryl sulphate, magnesium lauryl sulphate, fumaric acid, glyceryl palmitostearate, hydrogenated natural oils, zinc stearate, calcium stearate, silica, talc, stearic acid, paraffin and mixtures thereof, preferably polyethylene glycol.

9. The effervescent formulation according to claim 9, wherein the amount of polyethylene glycol is in the range of 0.1 to 15 %, preferably 0.1 to 10%, more preferably it is 0.1 to 2% by weight of total formulation.

10. The effervescent formulation according to any preceding claim comprising;
a) 2.0 - 80.0 % by weight of linagliptin
b) 0.1 - 15.0 % by weight of polyethylene glycol
c) 2.0 - 90.0 % by weight of sodium glycine carbonate
d) 2.0 - 90.0 % by weight of fumaric acid
e) 5.0 - 90.0 % by weight of lactose
f) 0.1 - 0.2 % by weight of sweetener
g) 0.1 - 5.0 % by weight of aromatic agent

11. The effervescent formulation according to any preceding claim; is used by directly taking into mouth.

12. The effervescent formulation according to any preceding claim; is used by introducing the formulation into water to be administered.
